# EUROPÄISCHE PATENTANMELDUNG

(11) **EP 4 266 323 A2**
(43) Veröffentlichungstag der Anmeldung: **25.10.2023**
(21) Anmeldenummer: 23190920.1
(22) Anmeldetag: 13.01.2020
(51) Int. Cl.: G16H 40/20

(54) **REKRUTIERUNG VON PATIENTEN FÜR ARZNEIMITTELSTUDIEN**

(30) Priorität: 18.01.2019 EP 19152557
(62) Teilanmeldung aus: 20700168.6
(71) Anmelder: Bayer Aktiengesellschaft, 51373 Leverkusen (DE)
(72) Erfinder: MÜLLER, Christian Johannes, 51375 Leverkusen (DE)
(74) Vertreter: BIP Patents

(57) **Zusammenfassung**

Die vorliegende Erfindung befasst sich mit der Rekrutierung von Patienten für eine oder mehrere Arzneimittelstudien. Gegenstände der vorliegenden Erfindung sind ein Verfahren, ein System und ein Computerprogrammprodukt zur Rekrutierung von Patienten für eine oder mehrere Arzneimittelstudien mit Hilfe eines individuellen Erkennungsmerkmals, das auf der Verpackung eines Arzneimittels aufgebracht ist.

## Beschreibung

Die vorliegende Erfindung befasst sich mit der Rekrutierung von Patienten für eine oder mehrere Arzneimittelstudien. Gegenstände der vorliegenden Erfindung sind ein Verfahren, ein System und ein Computerprogrammprodukt zur Rekrutierung von Patienten für eine oder mehrere Arzneimittelstudien mit Hilfe eines individuellen Erkennungsmerkmals, das auf der Verpackung eines Arzneimittels aufgebracht ist.

Unter dem Begriff Arzneimittelstudien werden Studien verstanden, in denen interventionell oder nicht-interventionell Wirkungen von Arzneimitteln erforscht oder nachgewiesen werden, und sich vom Nutzen-Risikoverhältnis oder der Unbedenklichkeit oder Wirksamkeit der Arzneimittel überzeugt wird. Arzneimittelstudien sind eine Voraussetzung für die Zulassung neuer Arzneimittel. In verschiedenen Phasen wird geprüft, ob das Arzneimittel die Anforderungen und die Erwartungen erfüllt, in Phase I an gesunden Freiwilligen (Probanden) und in den Phasen II und III an Patienten.

Nachdem die zuständige Zulassungsbehörde den Zulassungsbescheid erteilt hat, darf der pharmazeutische Unternehmer das Arzneimittel wie im Zulassungsbescheid beschrieben in den Verkehr bringen. Aber auch nach ihrer Zulassung sind viele Arzneimittel Gegenstand von Studien. In so genannten Phase-IV-Studien werden weitere Informationen zur Verträglichkeit, zur therapeutischen Effizienz, zur Produktprofilierung und zur Bearbeitung spezieller Fragestellungen (z.B. Langzeitverträglichkeit, seltene Nebenwirkungen usw.) gewonnen. Wird ein zugelassenes Arzneimittel in einer neuen Indikation geprüft, tritt man definitionsgemäß wieder in die Phase II oder III ein; manchmal werden solche Studien auch als Phase-V-Studien bezeichnet.

Üblicherweise bezahlt ein Sponsor (üblicherweise der pharmazeutische Unternehmer) alle Teilnehmer, die an der Studie mitwirken. Dazu zählen zumindest die teilnehmenden Patienten und ein Arzt (ein Versuchsleiter), dem die Beaufsichtigung der Patienten unterliegt.

So genannte Vertragsforschungsorganisationen (engl. Contract Research Organisation, CRO) bieten ihre Dienste zwischen dem Sponsor einerseits und dem Versuchsleiter und den Patienten andererseits an. Die CRO übernehmen häufig die gesamte Verwaltung der Studie, einschließlich aller notwendigen Dienste, darunter zum Beispiel die Entwicklung des Studienprotokolls, die Rekrutierung von Patienten und Versuchsleitern und/oder Versuchsstandorten, den Vertragsschluss mit den Teilnehmern, die Beaufsichtigung der Ausführung der Studie, das Sammeln und Auswerten von Daten, das Kanalisieren der Bezahlung vom Sponsor zu den Teilnehmern usw.

Beim Rekrutieren der Patienten greifen CROs auf interne Patientendatenbanken zu oder potenzielle Patienten werden von Ärzten angesprochen. Es gibt auch technische Systeme zur Unterstützung der Patientenrekrutierung (siehe z.B. US2008177573, WO2004092912, WO2015127245, WO2018224156).

Die Identifikation von geeigneten Patienten ist jedoch noch immer zeit- und kostenaufwändig. Viele Studien scheitern daran, dass die notwendige Zahl an geeigneten Patienten nicht zustande kommt. Häufig fehlt es nicht an der Bereitschaft zur Teilnahme, sondern am Informationsfluss in Richtung der in Frage kommenden Patienten.

Ausgehend vom beschriebenen Stand der Technik stellt sich einem Fachmann die technische Aufgabe, eine Lösung für die Rekrutierung von Patienten für Arzneimittelstudien, insbesondere für Phase-IV-Studien, bereitzustellen, bei der eine große Zahl an potenziellen und geeigneten Patienten angesprochen wird, bei der sich die Patienten auf einfache Weise für eine Studie anmelden können, und bei der Patienteninformationen auf einfache und schnelle Weise abgefragt und dem Sponsor und/oder der Vertragsforschungsorganisation zur Verfügung gestellt werden können.

Diese Aufgabe wird durch die Gegenstände der unabhängigen Patentansprüche gelöst. Bevorzugte Ausführungsformen finden sich in den abhängigen Patentansprüchen sowie in der vorliegenden Beschreibung und in den Figuren.

Ein erster Gegenstand der vorliegenden Erfindung ist ein Verfahren umfassend die folgenden Schritte:
- Eingeben von Daten eines individuellen Erkennungsmerkmals, das auf einer Einzelpackung eines Arzneimittels angebracht ist, durch einen potenziellen Teilnehmer einer Arzneimittelstudie in einen Computer,
- Identifizieren einer oder mehrerer Arzneimittelstudien anhand der eingegebenen Daten,
- Anzeigen der einer oder der mehreren Arzneimittelstudien gegenüber dem potenziellen Teilnehmer.

Ein weiterer Gegenstand der vorliegenden Erfindung ist ein System umfassend
- eine Eingabeeinheit,
- eine Ausgabeeinheit,
- eine Steuereinheit und
- eine Datenbank,

wobei die Steuereinheit konfiguriert ist, die Eingabeeinheit zu veranlassen, Daten eines individuellen Erkennungsmerkmals, das auf einer Einzelpackung eines Arzneimittels angebracht ist, von einem potenziellen Teilnehmer einer Arzneimittelstudie zu empfangen,
wobei die Steuereinheit konfiguriert ist, anhand der empfangenen Daten eine oder mehrere Arzneimittelstudien in der Datenbank zu identifizieren,
wobei die Steuereinheit konfiguriert ist, die Ausgabeeinheit zu veranlassen, Informationen zu der einen oder den mehreren identifizierten Arzneimittelstudien gegenüber dem potenziellen Teilnehmer anzuzeigen.

Ein weiterer Gegenstand der vorliegenden Erfindung ist ein Computerprogrammprodukt umfassend einen Datenträger und Programmcode, der auf dem Datenträger gespeichert ist, und der einen Computer, in dessen Arbeitsspeicher der Programmcode geladen ist, dazu veranlasst, die folgenden Schritte auszuführen:
- Empfangen von Daten eines individuellen Erkennungsmerkmals, das auf einer Einzelpackung eines Arzneimittels aufgebracht ist,
- Identifizieren einer oder mehrerer Arzneimittelstudien anhand der empfangenen Daten,
- Anzeigen von Informationen über die eine oder die mehreren Arzneimittelstudien gegenüber einem potenziellen Teilnehmer der einen oder der mehreren Arzneimittelstudien.

Ein weiterer Gegenstand der vorliegenden Erfindung ist die Verwendung eines individuellen Erkennungsmerkmals, das auf einer Einzelpackung eines Arzneimittels angebracht ist, zur Rekrutierung von Teilnehmern für eine Arzneimittelstudie, dadurch gekennzeichnet, dass ein potenzieller Teilnehmer Daten des individuellen Erkennungsmerkmals in einen Computer eingibt, anhand der Daten eine oder mehrere Arzneimittelstudien identifiziert wird/werden, und dem potenziellen Teilnehmer Informationen zu der einen oder den mehreren Arzneimittelstudien angezeigt werden.

Die Erfindung wird nachstehend näher erläutert, ohne zwischen den Erfindungsgegenständen (Verfahren, System, Computerprogrammprodukt, Verwendung) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt, Verwendung) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden können; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Mit der vorliegenden Erfindung können Teilnehmer für eine Arzneimittelstudie einfach und effizient rekrutiert werden.

Unter dem Begriff "Teilnehmer" wird ein Mensch verstanden, der ärztliche Dienstleistungen in Anspruch nimmt (Patient) und freiwillig an einer Arzneimittelstudie teilnimmt. Der Begriff "Teilnehmer" schließt weibliche Teilnehmerinnen mit ein; das Gleiche gilt für die Begriffe "potenzieller Teilnehmer" und "Patient".

Unter dem Begriff "potenzieller Teilnehmer" wird ein Patient verstanden, der prüft und/oder für den geprüft wird, ob er an einer Arzneimittelstudie teilnehmen kann. Ist er selbst bereit, an einer Arzneimittelstudie teilzunehmen und erfüllt er die Voraussetzungen zur Teilnahme an der Arzneimittelstudie, kann er sich für die Arzneimittelstudie registrieren. Durch die Registrierung wird er zum Teilnehmer. Die Registrierung umfasst seine informierte Einwilligung (engl. *informed consent*)*.*

Unter dem Begriff "Arzneimittelstudie" wird eine Studie verstanden, in der interventionell oder nicht-interventionell Wirkungen eines oder mehrerer Arzneimittel erforscht oder nachgewiesen werden und/oder sich vom Nutzen-Risikoverhältnis und/oder der Unbedenklichkeit und/oder Wirksamkeit des Arzneimittels / der Arzneimittel überzeugt wird.

Vorzugsweise handelt es sich bei der Arzneimittelstudie, für die Patienten rekrutiert werden sollen, um eine nicht-interventionelle Studie. Eine "nicht-interventionelle Studie" ist eine Untersuchung, in deren Rahmen Erkenntnisse aus der Behandlung von Personen mit Arzneimitteln anhand epidemiologischer Methoden analysiert werden; dabei folgt die Behandlung einschließlich der Diagnose und Überwachung nicht einem vorab festgelegten Prüfplan, sondern ausschließlich der ärztlichen Praxis; soweit es sich um ein zulassungspflichtiges oder genehmigungspflichtiges Arzneimittel handelt, erfolgt dies ferner gemäß den in der Zulassung oder der Genehmigung festgelegten Angaben für seine Anwendung. Vorzugsweise handelt es sich bei der Arzneimittelstudie, für die Patienten rekrutiert werden sollen, um eine Phase-IV-Studie.

Unter dem Begriff "Arzneimittel" oder gleichbedeutend "Medikament" wird ein Stoff oder eine Stoffzusammensetzung verstanden, der bzw. die zur Heilung oder zur Verhütung menschlicher Krankheiten bestimmt ist oder sich zur Beeinflussung physiologischer Funktionen eignet oder eine medizinische Diagnose ermöglicht. Vorzugsweise handelt es sich bei dem Arzneimittel, das Gegenstand der Studie ist, um ein bereits zugelassenes Arzneimittel, das von einem pharmazeutischen Unternehmer bereits in Verkehr gebracht worden ist und somit von einem Patienten z.B. in einer Apotheke erworben werden kann. Vorzugsweise handelt es sich um ein verschreibungspflichtiges Arzneimittel.

Die erfindungsgemäße Rekrutierung von Patienten für eine oder mehrere Arzneimittelstudien erfolgt mit Hilfe eines individuellen Erkennungsmerkmals, das auf einer Einzelpackung eines Arzneimittels angebracht ist.

Gemäß Art. 54a Abs. 1 der durch die sog. EU-Fälschungsschutzrichtlinie 2011/62/EU (FMD) geänderten Richtlinie 2001/83/EG sind zumindest verschreibungspflichtige Arzneimittel mit einem solchen individuellen Erkennungsmerkmal (engl.: *Unique Identifier*) zu kennzeichnen, das insbesondere eine Überprüfung der Echtheit und die Identifizierung von Einzelpackungen ermöglicht.

Die Einzelheiten hinsichtlich der Eigenschaften und technischen Spezifikationen des individuellen Erkennungsmerkmals sind in der delegierten Verordnung (EU) 2016/161 der Kommission vom 2. Oktober 2015 festgelegt und am 9. Februar 2016 im Europäischen Amtsblatt veröffentlicht.

Gemäß Art. 4 der delegierten Verordnung umfasst das individuelle Erkennungsmerkmal die folgenden Datenelemente: Produktcode, Seriennummer, Chargenbezeichnung und Verfalldatum. Zusätzlich stehen diese Angaben auch in Klarschrift auf der Einzelpackung.

Über den Produktcode kann das Arzneimittel spezifiziert werden, d.h. es kann festgestellt werden, um welches Arzneimittel es sich handelt.

Die Kombination aus Produktcode und Seriennummer ist weltweit für jede Arzneimittelpackung (zumindest für einen definierten Zeitraum) eindeutig.

Die genannten Datenelemente (Produktcode, Seriennummer, Chargenbezeichnung und Verfalldatum) sind in Form eines maschinenlesbaren zweidimensionalen Codes (z.B. Data Matrix Code) auf einer Verpackung des Arzneimittels aufgedruckt. Die Verpackung und das darin befindliche Arzneimittel bilden eine, durch das individuelle Erkennungsmerkmal individualisierte Einzelpackung.

Die Datenelemente des individuellen Erkennungsmerkmals (zumindest der Produktcode und die Seriennummer) werden zusätzlich in einer Datenbank (Verifikationsdatenbank) gespeichert. Wird eine Einzelpackung z.B. in einer Apotheke an einen Patienten abgegeben, muss der Apotheker zunächst den zweidimensionalen Code mit einem Lesegerät auslesen. Dies löst eine Überprüfung von Produktcode und Seriennummer gegenüber der Verifikationsdatenbank aus; es wird geprüft, ob die Kombination von Produktcode und Seriennummer vorhanden ist und ob eine Einzelpackung mit dieser Kombination bereits als "abgegeben" in der Verifikationsdatenbank vermerkt ist. Ist die Kombination von Produktcode und Seriennummer vorhanden, und ist die Einzelpackung noch nicht "abgegeben", darf der Apotheker sie an den Patienten abgeben. Wird die Einzelpackung nach positiver Rückmeldung abgegeben, wird der Status der Einzelpackung auf "abgegeben" gesetzt. Sollte nun eine zweite Einzelpackung mit identischer Kombination aus Produktcode und Seriennummer einer Verifizierung unterzogen werden, wird offenkundig, dass diese bereits abgegeben wurde. Sollte eine Einzelpackung der Verifizierung unterzogen werden, deren Kombination aus Produktcode und Seriennummer nicht in der Verifikationsdatenbank vorhanden ist, muss es sich um eine Fälschung handeln.

Weitere Anforderungen und Informationen zum individuellen Erkennungsmerkmal sind der delegierten Verordnung und den darin genannten Referenzen zu entnehmen, deren Inhalt durch Bezugnahme in diese Beschreibung aufgenommen wird.

Das individuelle Erkennungsmerkmal macht eine individuelle Einzelpackung erkennbar. Da üblicherweise eine individuelle Einzelpackung nur von einer einzigen Person verwendet wird (insbesondere dann, wenn es sich um ein verschreibungspflichtiges Arzneimittel handelt), kann das individuelle Erkennungsmerkmal verwendet werden, um den Besitzer der Einzelpackung, auf der das individuelle Erkennungsmerkmal aufgebracht ist, ebenfalls erkennbar (identifizierbar) zu machen.

Anhand des individuellen Erkennungsmerkmals kann also verifiziert werden, dass eine Person im Besitz des entsprechenden Arzneimittels ist, auf dessen Verpackung das individuelle Erkennungsmerkmal aufgebracht ist. Diese Person ist üblicherweise ein Patient, der das Arzneimittel einnimmt. Damit ist diese Person ein potenzieller Teilnehmer einer Arzneimittelstudie im Zusammenhang mit dem Arzneimittel.

Nur diejenige Person, die im Besitz des individuellen Erkennungsmerkmals ist, kann das individuelle Erkennungsmerkmal einsetzen. Damit ist das individuelle Erkennungsmerkmal auf der Einzelpackung quasi eine personengebundene Eintrittskarte, die für die Anmeldung der Person für eine oder mehrere Arzneimittelstudien verwendet werden kann.

Das individuelle Erkennungsmerkmal oder ein davon abgeleitetes individuelles Merkmal kann ferner als eindeutige Kennung der Person als Teilnehmer an einer oder an mehreren Arzneimittelstudien verwendet werden.

Das individuelle Erkennungsmerkmal kann dazu verwendet werden, anhand des Produktcodes das entsprechende Arzneimittel und ggf. die Indikation, für die das Arzneimittel zugelassen ist, zu ermitteln.

Anhand des ermittelten Arzneimittels und/oder der ermittelten Indikation kann geprüft werden, ob eine Arzneimittelstudie durchgeführt wird oder geplant ist, die das ermittelte Arzneimittel und/oder die ermittelte Indikation zum Gegenstand hat/haben.

Erfindungsgemäß wird einem potenziellen Teilnehmer einer Arzneimittelstudie in einem ersten Schritt (vorzugsweise vom Sponsor der Arzneimittelstudie) ein Portal zur Verfügung gestellt, über das sich der potenzielle Teilnehmer informieren kann, ob eine Arzneimittelstudie geplant ist, für die Teilnehmer gesucht werden und/oder an der er teilnehmen könnte.

Das Portal kann einem potenziellen Teilnehmer zum Beispiel als ein Webportal auf einem Computer zur Verfügung gestellt werden. Bei dem Computer kann es sich um den eigenen Computer des potenziellen Teilnehmers handeln. Der Computer kann zum Beispiel als ein Desktop-PC, portabler PC, Laptop, Notebook, Netbook, Tablet-PC oder Handheld (z.B. Smartphone) ausgeführt sein.

Das Webportal kann z.B. über eine spezifische Internetadresse mit einem Webbrowser aufgerufen werden. Denkbar ist auch, dass das Portal in Form einer Software-Applikation aufgerufen wird, wobei die Software-Applikation auf dem Computer installiert ist und ausgeführt werden kann.

Denkbar ist auch, dass das Portal über ein Terminal (Kiosk), das beispielsweise in einem Krankenhaus oder einer Apotheke aufgestellt ist, aufgerufen werden kann.

Denkbar ist auch, dass die in dieser Beschreibung beschriebenen Aufgaben, die ein Computer ausführt, auf mehrere Computer verteilt sind. Denkbar ist zum Beispiel, dass der potenzielle Teilnehmer über einen Computer verfügt, der über ein oder mehrere Netzwerke (zum Beispiel ein Mobilfunknetz, ein WLAN und/oder das Internet) mit einem Server verbunden ist, über den der Sponsor oder der Versuchsleiter verfügt. Über das Portal können Daten, die der potenzielle Teilnehmer auf seinem Computer eingibt, auf den Server übertragen werden.

In einem ersten Schritt wird ein potenzieller Teilnehmer, der im Besitz einer Einzelpackung eines Arzneimittels mit einem individuellen Erkennungsmerkmal ist, aufgefordert, Daten des individuellen Erkennungsmerkmals über das Portal in den Computer einzugeben.

Bei den einzugebenden Daten handelt es sich mindestens um den Produktcode und die Seriennummer. Die Daten können von dem Nutzer als alphanumerische Zeichen beispielsweise mittels eines Eingabegeräts wie einer Tastatur, einer Maus oder einem berührungsempfindlichen Bildschirm (Touchscreen) eingegeben werden. Es ist ebenso denkbar, dass ein Mikrofon in Kombination mit einer Spracherkennungssoftware verwendet wird, um die Daten einzugeben.

Vorzugsweise verwendet der potenzielle Teilnehmer zur Eingabe der Daten ein Lesegerät, mit dem der maschinenlesbare zweidimensionale Code des individuellen Erkennungsmerkmal, der den Produktcode und die Seriennummer umfasst, automatisiert eingelesen werden kann. Das Lesegerät kann ein Bestandteil desjenigen Computers sein, über den das Portal aufgerufen wird, oder es kann mit diesem Computer verbunden sein (über eine Kabelverbindung oder kabellos z.B. über Bluetooth oder WLAN). Das Lesegerät ist konfiguriert, ein individuelles Erkennungsmerkmal auszulesen. Da das individuelle Erkennungsmerkmal gemäß der delegierten Verordnung (EU) 2016/161 als maschinenlesbarer zweidimensionaler Code (z.B. als QR-Code oder Data-Matrix-Code) auf der Verpackung des Arzneimittels aufgedruckt ist, kann das Lesegerät beispielsweise als Kamera ausgeführt sein. Eine Kamera ist üblicherweise ein Bestandteil von Smartphones und Tablet-Computern. Es kann sich bei dem Lesegerät aber auch um einen separaten Scanner / eine separate Kamera für zweidimensionale Codes handeln.

Der zweidimensionale Code wird von dem Lesegerät und/oder dem Computer interpretiert. Bei der Interpretation wird der zweidimensionale Code des individuellen Erkennungsmerkmals in alphanumerische Zeichen dekodiert.

In einem weiteren Schritt können aus den alphanumerischen Zeichen der Produktcode und die Seriennummer extrahiert werden.

In einem weiteren Schritt kann verifiziert werden, dass der potenzielle Teilnehmer im Besitz einer originalen Einzelpackung des Arzneimittels ist. Dazu kann geprüft werden, ob die Kombination aus Produktcode und Seriennummer in der Verifikationsdatenbank vorhanden ist. Ist die Kombination aus Produktcode und Seriennummer in der Verifikationsdatenbank vorhanden, gilt die Einzelpackung als "originale Einzelpackung". Ist die Kombination aus Produktcode und Seriennummer nicht in der Verifikationsdatenbank vorhanden, gilt die Einzelpackung als "Fälschung". Ferner kann der Status einer originalen Einzelpackung ermittelt werden. Ist der Status auf "abgegeben" gesetzt, gilt die Einzelpackung als "legal erworben". Ist der Status auf "nicht abgegeben" gesetzt, gilt sie als "nicht legal erworben".

In einer bevorzugten Ausführungsform erhält nur derjenige potenzielle Teilnehmer Informationen über eine oder mehrere Arzneimittelstudien und/oder es kann sich nur derjenige potenzielle Teilnehmer für eine Arzneimittelstudie anmelden und/oder registrieren, der im Besitz einer originalen Einzelpackung ist.

In einer bevorzugten Ausführungsform erhält nur derjenige potenzielle Teilnehmer Informationen über eine oder mehrere Arzneimittelstudien und/oder es kann sich nur derjenige potenzielle Teilnehmer für eine Arzneimittelstudie anmelden und/oder registrieren, der im Besitz einer legal erworbenen Einzelpackung ist.

In einer bevorzugten Ausführungsform erhält nur derjenige potenzielle Teilnehmer Informationen über eine oder mehrere Arzneimittelstudien und/oder es kann sich nur derjenige potenzielle Teilnehmer für eine Arzneimittelstudie anmelden und/oder registrieren, der im Besitz einer legal erworbenen und originalen Einzelpackung ist.

In einem weiteren Schritt kann anhand des Produktcodes das Arzneimittel spezifiziert werden. Ferner können anhand des Produktcodes die Darreichungsform, die Stärke, die Packungsgröße und die Verpackungsart des Arzneimittels ermittelt werden.

Die Informationen können über den Produktcode aus einer Datenbank (Arzneimitteldatenbank) ausgelesen werden, die Bestandteil des Computers sein kann oder die mit dem Computer über ein oder mehrere Netzwerke verbunden sein kann. Bei der Arzneimitteldatenbank und der Verifikationsdatenbank kann es sich um eine einzige Datenbank oder um verschiedene Datenbanken handeln.

Anhand des spezifizierten Arzneimittels und ggf. der weiteren Informationen zum Arzneimittel können in einer (weiteren) Datenbank (Arzneimittelstudiendatenbank) nach Arzneimittelstudien gesucht werden, für die Personen gesucht werden und/oder an denen eine Person, die das spezifizierte Arzneimittel einnimmt, teilnehmen könnte. Die Arzneimittelstudiendatenbank kann ein Bestandteil des Computers sein oder sie kann mit dem Computer über ein oder mehrere Netzwerke verbunden sein. Es kann sich bei der Arzneimittelstudiendatenbank und der Arzneimitteldatenbank um eine einzige Datenbank oder um verschiedene Datenbanken handeln.

Es ist beispielsweise denkbar, dass alle geplanten oder laufenden Arzneimittelstudien eines oder mehrerer Sponsoren, bei denen das spezifizierte Arzneimittel ein Untersuchungsgegenstand ist, aus der Arzneimittelstudiendatenbank ausgelesen werden.

Es ist denkbar, dass alle Arzneimittelstudien aus der Arzneimittelstudiendatenbank ausgelesen werden, bei denen Indikationen untersucht werden, für die das spezifizierte Arzneimittel eingesetzt wird oder eingesetzt werden könnte.

Es ist denkbar, dass für die Identifizierung der Arzneimittelstudie, für die der potenzielle Teilnehmer ein geeigneter Teilnehmer sein könnte, weitere Informationen über den potenziellen Teilnehmer und/oder seine Erkrankung herangezogen werden, wie beispielsweise sein Alter, Geschlecht, Körpergröße, Körpergewicht, Krankheitssymptome, diagnostizierte Erkrankung, Vorerkrankungen und/oder dergleichen. Vorzugsweise werden diese Patienteninformationen über das Portal abgefragt und vom potenziellen Teilnehmer eingegeben.

Die identifizierte(n) Arzneimittelstudie(n), an der/denen der potenzielle Teilnehmer teilnehmen könnte, wird/werden dem potenziellen Teilnehmer gegenüber angezeigt. Eine solche Anzeige kann zum Beispiel in Textform ggf. untermalt mit grafischen Elementen oder Bildern auf einem Bildschirm erfolgen. Der Bildschirm kann ein Bestandteil des Computers sein oder mit diesem verbunden sein. Auch eine Sprachausgabe über einen Lautsprecher ist denkbar.

Vorzugsweise kann sich der potenzielle Teilnehmer über das Portal für eine oder mehrere Arzneimittelstudien registrieren. Mit der Registrierung erklärt der potenzielle Teilnehmer sein Einverständnis für die Teilnahme an der Arzneimittelstudie und für die Verarbeitung der anfallenden Daten. Üblicherweise erteilt er sein Einverständnis durch eine sogenannte "Einwilligung nach erfolgter Aufklärung", auch "informierte Einwilligung" genannt (engl. *informed consent*). Diese informierte Einwilligung kann er beispielsweise durch eine digitale oder elektronische Signatur über das Portal erteilen. Denkbar ist auch, dass ihm über das Portal ein entsprechendes Schriftstück angezeigt wird, das er mit einem Drucker ausdrucken, unterzeichnen und per Post an den Sponsor oder Versuchsleiter senden kann, oder das er, nach Einlesen mit Hilfe eines Dokumentenscanners in den Computer, über das Portal auf den Server übertragen kann.

In einer Ausführungsform der vorliegenden Erfindung wird der Teilnehmer bei der Registrierung für eine Arzneimittelstudie mit einer individuellen Kennung verknüpft, die aus einer Kombination aus Produktcode und Seriennummer des individuellen Erkennungsmerkmals abgeleitet ist.

Gemäß der delegierten Verordnung (EU) 2016/161 umfasst das individuelle Erkennungsmerkmal eine numerische oder alphanumerische Folge von höchstens 20 Zeichen, generiert durch einen deterministischen oder nicht-deterministischen Randomisierungsalgorithmus (die Seriennummer). Anhand der Kombination aus dem Produktcode und der Seriennummer kann eine Einzelpackung eines Arzneimittels eindeutig identifiziert werden. Wird diese Kombination aus Produktcode und Seriennummer (oder eine aus der Kombination abgeleitete individuelle Kennung) mit dem (potenziellen) Teilnehmer verknüpft, der über die Einzelpackung verfügt, kann der (potenzielle) Teilnehmer eindeutig anhand dieser Kombination (oder einer aus der Kombination abgeleiteten individuellen Kennung) identifiziert werden. Dadurch ist es nicht erforderlich, personenbezogene Daten wie den Namen oder die Adresse des (potenziellen) Teilnehmers zu erfassen und zu speichern - was aus Datenschutzgründen vorteilhaft ist. Stattdessen kann eine eindeutige Zuordnung von Ergebnissen der Arzneimittelstudie zu einem Teilnehmer auf Basis der Kombination aus Produktcode und Seriennummer (oder einer aus der Kombination abgeleiteten individuellen Kennung) getroffen werden.

Bei der Kombination von Produktcode und Seriennummer kann es sich um die Abfolge Produktcode/Seriennummer, um die Abfolge Seriennummer/Produktcode, um eine Ineinanderschachtelung von Produktcode und Seriennummer oder um eine sonstige Verknüpfung von Produktcode und Seriennummer handeln. Denkbar ist auch, dass Produktcode und Seriennummer einem (mathematischen) Algorithmus unterzogen werden, der aus dem Produktcode und der Seriennummer eine eindeutige Kennung erzeugt. Denkbar ist beispielsweise die Erzeugung eines Hash-Wertes oder dergleichen. Denkbar ist auch, dass neben dem Produktcode und der Seriennummer weitere Daten in die Erzeugung einer eindeutigen Kennung einfließen. Denkbar ist ferner, dass nicht der gesamte Produktcode und/oder die gesamte Seriennummer zur Erzeugung einer eindeutigen Kennung verwendet wird/werden; es muss lediglich gewährleistet sein, dass die eindeutige Kennung einen Teilnehmer einer Arzneimittelstudie von anderen Teilnehmern unterscheidet.

In der Arzneimittelstudie kann der jeweilige Teilnehmer dann unter der Kombination aus Produktcode und Seriennummer oder unter der abgeleiteten eindeutigen Kennung geführt werden.

Die Kombination aus Produktcode und Seriennummer sowie die abgeleitete eindeutige Kennung sind vorzugsweise ein numerischer oder alphanumerischer Code (Zeichenkette).

Denkbar ist aber auch, dass der Teilnehmer in einer oder mehreren Arzneimittelstudien unter einer eindeutigen Kennung geführt wird, die bei der Installation und/oder dem erstmaligen Aufrufen des Portals und/oder bei der Registrierung für eine (oder die erste) Arzneimittelstudie erzeugt wird.

Vorzugsweise wird ein Teilnehmer bei verschiedenen Arzneimittelstudien unter derselben eindeutigen Kennung geführt.

Die Verwendung einer eindeutigen Kennung in mehreren Arzneimittelstudien hat den Vorteil, dass die Ergebnisse aus den mehreren Studien miteinander in Beziehung gesetzt und studienübergreifende Analysen durchgeführt werden können.

In einer bevorzugten Ausführungsform werden über das Portal Ergebnisse der Arzneimittelstudie erfasst. In der englischsprachigen Literatur wird der Begriff *"Patient Reported Outcome"* (Abk.: PRO) als Oberbegriff für viele verschiedene Konzepte zur Messung subjektiv empfundener Gesundheitszustände gebraucht. Die gemeinsame Grundlage dieser Konzepte ist, dass der Teilnehmer selbst seinen Zustand einschätzt und berichtet. Es ist beispielsweise denkbar, dass der Teilnehmer im Rahmen der Arzneimittelstudie regelmäßig und/oder zu definierten Zeitpunkten einen Fragebogen zur Selbsteinschätzung seiner Gesundheit ausfüllt. Vorzugsweise werden dem Teilnehmer Fragen oder auszufüllende Felder über das Portal angezeigt und er kann diese mit Hilfe eines Eingabegeräts (z.B. einer Tastatur, einer Maus, einem berührungsempfindlichen Bildschirm (Touchscreen), einem Mikrofon und/oder dergleichen) beantworten oder ausfüllen. Es ist denkbar, dass ein Chatbot eingesetzt wird, um dem Patienten die Eingabe von Informationen zu erleichtern.

Es ist denkbar, dass ein Teilnehmer über das Portal Anweisungen im Rahmen der Arzneimittelstudie erhält. Eine solche Anweisung kann beispielsweise die Aufforderung sein, eine Arzneimittelportion einzunehmen, eine Messung durchzuführen (Blutzuckermessung, Blutdruckmessung, Körperkerntemperaturmessung, Körpergewichtsmessung und/oder dergleichen), eine oder mehrere Fragen zu beantworten, eine Selbsteinschätzung zum Gesundheitszustand vorzunehmen und/oder dergleichen. Anweisungen können in Textform (ggf. untermalt mit grafischen Elementen oder Bildern) auf dem Computer des Teilnehmers angezeigt werden. Eine solche Anweisung kann beispielsweise durch einen Vibrationsalarm oder ein akustisches Signal begleitet sein, damit der Teilnehmer informiert ist, dass eine Anweisung eingetroffen ist.

Es ist denkbar, dass die Vergütung der Teilnahme eines Patienten an der Arzneimittelstudie über das Portal abgewickelt wird. Eine Vergütung kann beispielsweise in Form von Geld oder der Erstattung von Reisekosten, Lebensmitteln oder Lebensmittelgutscheinen, oder anderen Diensten erfolgen. Es ist denkbar, dass über das Portal Bankdaten für Überweisungen übermittelt werden, Gutscheincodes (Coupons) übermittelt werden, Kryptowährungen ausgezahlt werden, Rechnungen übertragen werden, Zeiten (zum Beispiel zum Ausfüllen von Fragebögen) erfasst werden und dergleichen.

Bevorzugte Ausführungsformen der vorliegenden Erfindung sind:
1. Ein Verfahren umfassend die folgenden Schritte:
   - Eingeben von Daten eines individuellen Erkennungsmerkmals, das auf einer Einzelpackung eines Arzneimittels angebracht ist, durch einen potenziellen Teilnehmer einer Arzneimittelstudie in einen Computer,
   - Identifizieren einer oder mehrerer Arzneimittelstudien, an denen der potenzielle Teilnehmer teilnehmen kann, anhand der eingegebenen Daten,
   - Anzeigen der einer oder der mehreren Arzneimittelstudien gegenüber dem potenziellen Teilnehmer.
2. Das Verfahren gemäß der Ausführungsform 1, umfassend die folgenden Schritte:
   - Extrahieren eines Produktcodes und einer Seriennummer aus den Daten des individuellen Erkennungsmerkmals,
   - Identifizieren eines Arzneimittels und/oder einer Indikation anhand des Produktcodes,
   - Identifizieren einer oder mehrerer Arzneimittelstudien, an denen der potenzielle Teilnehmer teilnehmen kann, anhand des identifizierten Arzneimittels und/oder der identifizierten Indikation.
3. Das Verfahren gemäß der Ausführungsform 1 oder 2, umfassend die folgenden Schritte:
   - Extrahieren eines Produktcodes und einer Seriennummer aus den Daten des individuellen Erkennungsmerkmals,
   - Verifizieren, dass es sich bei Einzelpackung um eine originale und/oder legal erworbene Einzelpackung handelt, anhand des Produktcodes und der Serienummer.
4. Das Verfahren gemäß der Ausführungsform 3, wobei dem potenziellen Teilnehmer eine oder mehrere Arzneimittelstudien, an denen er teilnehmen kann, nur dann angezeigt wird/werden, wenn es sich bei der Einzelpackung um eine originale und/oder legal erworbene Einzelpackung handelt.
5. Das Verfahren gemäß einer der Ausführungsformen 1 bis 4, wobei der potenzielle Teilnehmer einen maschinenlesbaren zweidimensionalen Code des individuellen Erkennungsmerkmals mit einem Lesegerät für maschinenlesbare zweidimensionale Codes erfasst und der erfasste Code vom Lesegerät auf den Computer übertragen wird und der Computer aus dem erfassten Code einen Produktcode und eine Seriennummer extrahiert.
6. Das Verfahren gemäß einer Ausführungsformen 1 bis 5, umfassend die Schritte:
   - Eingeben von Patienteninformationen durch den potenziellen Teilnehmer in den Computer,
   - Identifizieren einer oder mehrerer Arzneimittelstudien unter Verwendung der Patienteninformationen.
7. Das Verfahren gemäß einer Ausführungsformen 1 bis 6, umfassend den Schritt:
   - Bereitstellen eines Portals zum Anmelden von Teilnehmern an einer Arzneimittelstudie auf dem Computer, wobei der Computer durch den potenziellen Teilnehmer bedient wird, wobei der potenzielle Teilnehmer über das Portal Daten des individuellen Erkennungsmerkmals und Patienteninformationen in den Computer eingibt, und dem potenziellen Teilnehmer über das Portal Informationen zu einer oder zu mehreren Arzneimittelstudien, an denen er teilnehmen kann, angezeigt werden.
8. Das Verfahren gemäß der Ausführungsform 7, umfassend die Schritte:
   - Registrieren des potenziellen Teilnehmers als einen Teilnehmer einer identifizierten Arzneimittelstudie,
   - Erfassen von Ergebnissen der Arzneimittelstudie, für die der Teilnehmer registriert ist, über das Portal, insbesondere durch Eingabe von Informationen zum Gesundheitszustand des Teilnehmers durch den Teilnehmer.
9. Das Verfahren gemäß einer der Ausführungsformen 1 bis 8, umfassend die Schritte:
   - Eingeben von Daten eines individuellen Erkennungsmerkmals auf einer Einzelpackung eines Arzneimittels durch einen potenziellen Teilnehmer einer Arzneimittelstudie in einen Computer,
   - Verifizieren, dass der potenzielle Teilnehmer im Besitz einer originalen Einzelpackung des Arzneimittels ist, durch Prüfen der Echtheit des individuellen Erkennungsmerkmals anhand der eingegebenen Daten,
   - Identifizieren einer oder mehrerer Arzneimittelstudien, an denen der potenzielle Teilnehmer teilnehmen kann, anhand der eingegebenen Daten,
   - Anzeigen der einer oder der mehreren identifizierten Arzneimittelstudien gegenüber dem potenziellen Teilnehmer.
10. Das Verfahren gemäß einer der Ausführungsformen 1 bis 9, umfassend die Schritte:
   - Erfassen eines maschinenlesbaren, zweidimensionalen Codes eines individuellen Erkennungsmerkmals auf einer Einzelpackung eines Arzneimittels mit Hilfe eines Lesegerätes durch einen potenziellen Teilnehmer einer Arzneimittelstudie an einem ersten Computer
   - Ermitteln eines Produktcodes und einer Seriennummer aus dem zweidimensionalen Code
   - Übertragen des Produktcodes und der Seriennummer vom ersten Computer an einen zweiten Computer
   - Ermitteln eines Arzneimittels und/oder einer Indikation anhand des übertragenen Produktcodes und mit Hilfe einer Arzneimitteldatenbank, in der Informationen über das Arzneimittel unter dem Produktcode gespeichert sind
   - Identifizieren einer oder mehrerer Arzneimittelstudien, bei denen das ermittelte Arzneimittel und/oder die ermittelte Indikation ein Untersuchungsgegenstand sind, mit Hilfe einer Arzneimittelstudiendatenbank
   - Übertragen der einen oder mehreren identifizierten Arzneimittelstudien vom zweiten Computer an den ersten Computer
   - Anzeigen der übertragenen einen oder mehreren identifizierten Arzneimittelstudien am ersten Computer gegenüber dem potenziellen Teilnehmer.
11. Ein System umfassend
   - eine Eingabeeinheit,
   - eine Ausgabeeinheit,
   - eine Steuereinheit und
   - eine Datenbank,

   wobei die Steuereinheit konfiguriert ist, die Eingabeeinheit zu veranlassen, Daten eines individuellen Erkennungsmerkmals, das auf einer Einzelpackung eines Arzneimittels angebracht ist, von einem potenziellen Teilnehmer einer Arzneimittelstudie zu empfangen,
   wobei die Steuereinheit konfiguriert ist, anhand der empfangenen Daten eine oder mehrere Arzneimittelstudien in der Datenbank zu identifizieren,
   wobei die Steuereinheit konfiguriert ist, die Ausgabeeinheit zu veranlassen, Informationen zu der einen oder den mehreren identifizierten Arzneimittelstudien gegenüber dem potenziellen Teilnehmer anzuzeigen.
12. Das System gemäß der Ausführungsform 11 umfassend
   - einen ersten Computer mit einem Lesegerät und einer Ausgabeeinheit, und
   - einen zweiten Computer, der mit einer Arzneimitteldatenbank, einer Arzneimittelstudiendatenbank und einer Verifikationsdatenbank verbunden ist,
   wobei der erste Computer konfiguriert ist,
   - einen maschinenlesbaren zweidimensionalen Code eines individuellen Erkennungsmerkmals mit Hilfe des Lesegeräts zu erfassen,
   - aus dem Code einen Produktcode und eine Seriennummer zu extrahieren,
   - den Produktcode und die Seriennummer über ein Netzwerk an den zweiten Computer zu übermitteln,
   wobei der zweite Computer konfiguriert ist,
   - den Produktcode und die Seriennummer über das Netzwerk von dem ersten Computer zu empfangen,
   - zu verifizieren, dass in der Verifikationsdatenbank Informationen zu einer Einzelpackung eines Arzneimittels gespeichert sind, wobei der Einzelpackung der Produktcode und der Seriennummer zugeordnet sind,
   - anhand des Produktcodes ein Arzneimittel und/oder eine Indikation in der Arzneimitteldatenbank zu identifizieren,
   - anhand des identifizierten Arzneimittels und/oder der identifizierten Indikation ein oder mehrere Arzneimittelstudien in der Arzneimittelstudiendatenbank zu identifizieren,
   - Informationen zu der einen oder den mehreren identifizierten Arzneimittelstudien über das Netzwerk an den ersten Computer zu übermitteln,
   wobei der erste Computer konfiguriert ist,
   - die Informationen zu der einen oder den mehreren identifizierten Arzneimittelstudien über das Netzwerk von dem zweiten Computer zu empfangen,
   - die Informationen zu der einen oder den mehreren identifizierten Arzneimittelstudien über die Ausgabeeinheit anzuzeigen.
13. Das System gemäß der Ausführungsform 12,
   wobei der erste Computer konfiguriert ist, Patienteninformationen und/oder Informationen zur Selbsteinschätzung des Gesundheitszustands eines Teilnehmers einer Arzneimittelstudie über die Eingabeeinheit zu empfangen und an den zweiten Computer über ein Netzwerk zu übermitteln.
14. Ein Computerprogrammprodukt umfassend einen Datenträger und Programmcode, der auf dem Datenträger gespeichert ist, und der einen Computer, in dessen Arbeitsspeicher der Programmcode geladen ist, dazu veranlasst, die folgenden Schritte auszuführen:
   - Empfangen von Daten eines individuellen Erkennungsmerkmals, das auf einer Einzelpackung eines Arzneimittels aufgebracht ist,
   - Identifizieren einer oder mehrerer Arzneimittelstudien anhand der empfangenen Daten,
   - Anzeigen von Informationen über die eine oder die mehreren Arzneimittelstudien gegenüber einem potenziellen Teilnehmer der einen oder der mehreren Arzneimittelstudien.
15. Eine Verwendung eines individuellen Erkennungsmerkmals, das auf einer Einzelpackung eines Arzneimittels angebracht ist, zur Rekrutierung von Teilnehmern für eine Arzneimittelstudie, dadurch gekennzeichnet, dass ein potenzieller Teilnehmer Daten des individuellen Erkennungsmerkmals in einen Computer eingibt, anhand der Daten eine oder mehrere Arzneimittelstudien identifiziert werden, an der/denen der potenzielle Teilnehmer teilnehmen kann und dem potenziellen Teilnehmer Informationen zu der einen oder den mehreren Arzneimittelstudien angezeigt werden.

Die Erfindung wird nachfolgend anhand von Figuren näher erläutert, ohne die Erfindung auf die in den Figuren gezeigten Merkmale und Merkmalskombinationen beschränken zu wollen.

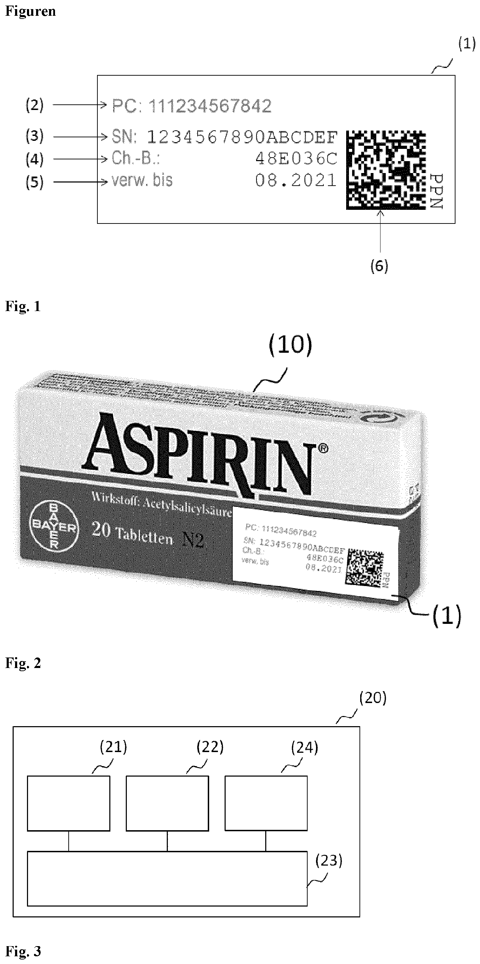

Figur 1 zeigt schematisch ein Beispiel eines individuellen Erkennungsmerkmals gemäß der delegierten Verordnung (EU) 2016/161 der Europäischen Kommission.

Das individuelle Erkennungsmerkmal (1) umfasst alphanumerische Zeichen (Klarschrift) wie einen Produktcode (2), eine Seriennummer (3), eine Chargenbezeichnung (4) und ein Verfallsdatum (5).

Der Produktcode (1) spezifiziert das Arzneimittel. Bei in Deutschland verkehrsfähigen Arzneimitteln wird als Produktcode die so genannte *Pharmacy Product Number* (PPN) herangezogen.

Das in Fig. 1 gezeigte individuelle Erkennungsmerkmal (1) umfasst ferner einen Data-Matrix-Code (6), in dem der Produktcode (2), die Seriennummer (3), die Chargenbezeichnung (4) und das Verfallsdatum (5) maschinenlesbar kodiert sind.

Figur 2 zeigt eine Einzelpackung (10) eines Arzneimittels, auf der das individuelle Erkennungsmerkmal (1) aus Figur 1 aufgedruckt ist.

Figur 3 zeigt schematisch eine Ausführungsform des erfindungsgemäßen Systems. Das System (20) umfasst eine Eingabeeinheit (21), eine Ausgabeeinheit (22), eine Steuereinheit (23) und eine Datenbank (24).

Die Steuereinheit (23) ist konfiguriert, die Eingabeeinheit (21) zu veranlassen, Daten eines individuellen Erkennungsmerkmals, das auf einer Einzelpackung eines Arzneimittels aufgebracht ist, von einem potenziellen Teilnehmer einer Arzneimittelstudie zu empfangen, und anhand der empfangenen Daten eine oder mehrere Arzneimittelstudien in der Datenbank (24) zu identifizieren, und die Ausgabeeinheit (22) zu veranlassen, Informationen zu der einen oder den mehreren identifizierten Arzneimittelstudien dem potenziellen Teilnehmer gegenüber anzuzeigen.

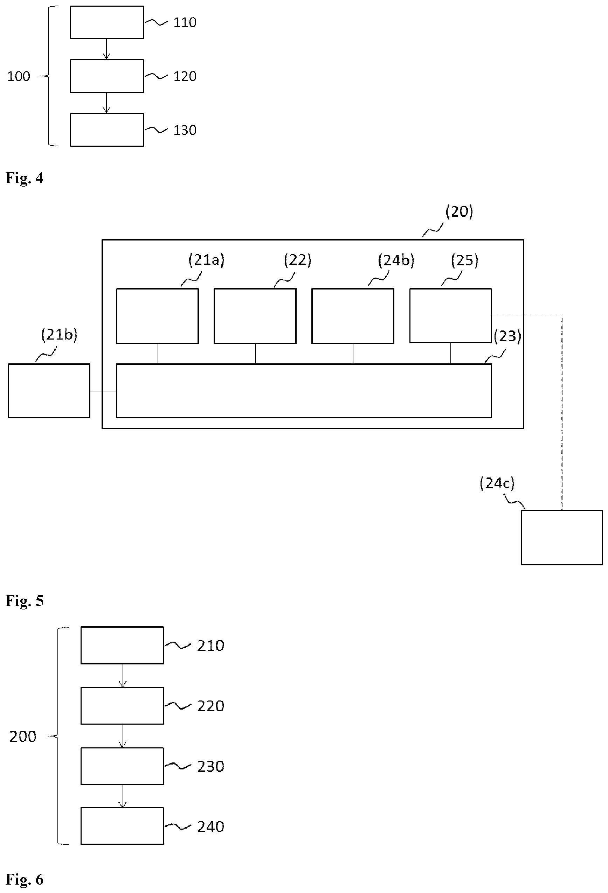

Figur 4 zeigt schematisch in einem Ablaufdiagramm eine Ausführungsform des erfindungsgemäßen Verfahrens, die mit dem in Figur 3 gezeigten System ausgeführt werden kann.

Das Verfahren (100) umfasst die Schritte:
(110) Eingeben von Daten eines individuellen Erkennungsmerkmals, das auf einer Einzelpackung eines Arzneimittels angebracht ist, durch einen potenziellen Teilnehmer einer Arzneimittelstudie in einen Computer,
(120) Identifizieren einer oder mehrerer Arzneimittelstudien, für die Teilnehmer gesucht werden und/oder an denen der potenzielle Teilnehmer teilnehmen kann, anhand der eingegebenen Daten,
(130) Anzeigen der einer oder der mehreren Arzneimittelstudien gegenüber dem potenziellen Teilnehmer.

Figur 5 zeigt schematisch eine weitere Ausführungsform des erfindungsgemäßen Systems. Das System (20) umfasst eine Eingabeeinheit (21a), ein Lesegerät (21b) für maschinenlesbare zweidimensionale Codes, eine Ausgabeeinheit (22), eine Steuereinheit (23), eine Arzneimittelstudiendatenbank (24b) und eine Sende- und Empfangseinheit (25).

Das Lesegerät (21b) wird üblicherweise von einem potenziellen Teilnehmer einer Arzneimittelstudie verwendet, um den maschinenlesbaren zweidimensionalen Code eines individuellen Erkennungsmerkmals auf einer Einzelpackung eines Arzneimittels zu erfassen. Die Steuereinheit (23) ist konfiguriert, die erfassten Daten vom Lesegerät (21b) zu empfangen. Je nach Ausgestaltung kann es sich bei den erfassten Daten um eine digitale Bildaufnahme des zweidimensionalen Codes oder um die daraus dekodierten alphanumerischen Daten (Produktcode, Seriennummer und dergleichen) handeln. Handelt es sich um eine digitale Bildaufnahme des zweidimensionalen Codes, so ist die Steuereinheit (23) konfiguriert, den zweidimensionalen Code zu dekodieren.

Über die Eingabeeinheit (21a) können Steuerbefehle und/oder weitere Daten eingegeben werden.

Die Steuereinheit (23) ist konfiguriert, aus den vom Lesegerät (21b) empfangenen Daten den Produktcode und die Seriennummer zu extrahieren. Die Steuereinheit (23) ist konfiguriert, zu verifizieren, dass der potenzielle Teilnehmer im Besitz einer originalen Einzelpackung des Arzneimittels ist. Dazu verifiziert die Steuereinheit (23), dass die Kombination aus Produktcode und Seriennummer in der Verifikationsdatenbank (24c) vorhanden ist. Die Steuereinheit (23) kann ferner konfiguriert sein, zu verifizieren, dass die originale Einzelpackung legal erworben worden ist. Dazu verifiziert die Steuereinheit (23), dass für die Einzelpackung in der Verifikationsdatenbank (24c) der Status "abgegeben" eingetragen ist.

Die Steuereinheit (23) ist ferner konfiguriert, anhand der vom Lesegerät empfangenen Daten (insbesondere anhand des Produktcodes) eine oder mehrere Arzneimittelstudien in der Arzneimittelstudiendatenbank (24b) zu identifizieren, und die Ausgabeeinheit (22) zu veranlassen, Informationen zu der einen oder den mehreren identifizierten Arzneimittelstudien dem potenziellen Teilnehmer gegenüber anzuzeigen.

Figur 6 zeigt schematisch in einem Ablaufdiagramm eine Ausführungsform des erfindungsgemäßen Verfahrens, die mit dem in Figur 5 gezeigten System ausgeführt werden kann.

Das Verfahren (200) umfasst die Schritte:
(210) Eingeben von Daten eines individuellen Erkennungsmerkmals, das auf einer Einzelpackung eines Arzneimittels angebracht ist, durch einen potenziellen Teilnehmer einer Arzneimittelstudie in einen Computer mit Hilfe eines Lesegeräts für maschinenlesbare zweidimensionale Codes,
(220) Verifizieren, dass der potenzielle Teilnehmer im Besitz einer originalen Einzelpackung des Arzneimittels ist, durch Prüfen der Echtheit des individuellen Erkennungsmerkmals anhand der eingegebenen Daten,
(230) Identifizieren einer oder mehrerer Arzneimittelstudien, für die Teilnehmer gesucht werden und/oder an denen der potenzielle Teilnehmer teilnehmen kann, anhand der eingegebenen Daten,
(240) Anzeigen der einer oder der mehreren identifizierten Arzneimittelstudien gegenüber dem potenziellen Teilnehmer.

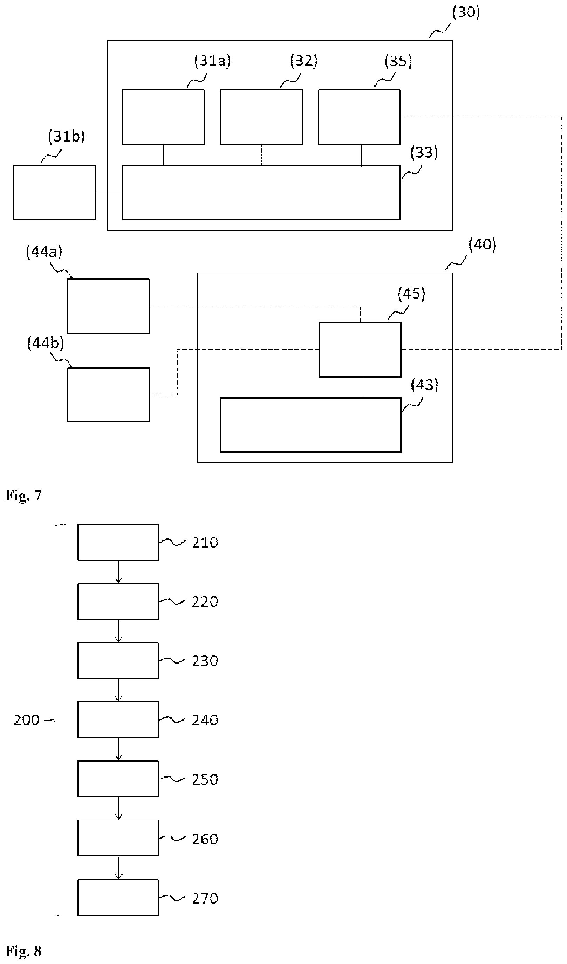

Figur 7 zeigt schematisch eine weitere Ausführungsform des erfindungsgemäßen Systems. Das System (20) umfasst einen ersten Computer (30), einen zweiten Computer (40), ein Lesegerät (31b) für zweidimensionale, maschinenlesbare Codes, eine Arzneimitteldatenbank (44a) und eine Arzneimittelstudiendatenbank (44b).

Der erste Computer (30) umfasst eine Eingabeeinheit (31a), eine Ausgabeeinheit (32), eine Steuereinheit (33) und eine Sende- und Empfangseinheit (35).

Der zweite Computer (40) umfasst eine Steuereinheit (43) und eine Sende- und Empfangseinheit (45).

Der erste Computer (30) und der zweite Computer (40) sind über ein Netzwerk oder über mehrere Netzwerke miteinander verbunden (dargestellt durch die gestrichelte Linie zwischen der Sende- und Empfangseinheit (35) des ersten Computers (30) und der Sende- und Empfangseinheit (45) des zweiten Computers (40)).

Der zweite Computer (40) ist über ein Netzwerk oder über mehrere Netzwerke mit der Arzneimitteldatenbank (44a) und der Arzneimittelstudiendatenbank (44b) verbunden (dargestellt durch die gestrichelten Linien zwischen der Sende- und Empfangseinheit (45) des zweiten Computers (40) und der Arzneimitteldatenbank (44a) beziehungsweise der Arzneimittelstudiendatenbank (44b)).

Das Lesegerät (31b) ist mit dem ersten Computer (30) verbunden und wird üblicherweise von einem potenziellen Teilnehmer einer Arzneimittelstudie verwendet, um den maschinenlesbaren zweidimensionalen Codes eines individuellen Erkennungsmerkmals auf einer Einzelpackung eines Arzneimittels zu erfassen und die erfassten Daten an den ersten Computer (30) zu übermitteln. Die Steuereinheit (33) des ersten Computers (30) ist konfiguriert, die erfassten Daten vom Lesegerät (31b) zu empfangen. Je nach Ausgestaltung kann es sich bei den erfassten Daten um eine digitale Bildaufnahme des zweidimensionalen Codes oder um die daraus dekodierten alphanumerischen Daten (Produktcode, Seriennummer und dergleichen) handeln. Handelt es sich um eine digitale Bildaufnahme des zweidimensionalen Codes, so ist die Steuereinheit (33) des ersten Computers (30) konfiguriert, den zweidimensionalen Code zu dekodieren.

Über die Eingabeeinheit (31a) des ersten Computers (30) können Steuerbefehle und/oder weitere Daten (z.B. Patientendaten und/oder Informationen zur Selbsteinschätzung des Gesundheitszustands) in den Computer (30) eingegeben werden.

Über die Ausgabeeinheit (32) des ersten Computers (30) können Informationen gegenüber einem potenziellen Teilnehmer ausgegeben werden.

Über die Sende- und Empfangseinheit (35) des ersten Computers (30) können Daten vom ersten Computer (30) an den zweiten Computer (40) übermittelt werden und es können Daten vom zweiten Computer (40) empfangen werden.

Über die Sende- und Empfangseinheit (45) des zweiten Computers (40) können Daten vom ersten Computer (30) empfangen werden und es können Daten vom zweiten Computer (40) an den ersten Computer (30) übermittelt werden. Ferner können über die Sende- und Empfangseinheit (45) des zweiten Computers (40) Daten aus der Arzneimitteldatenbank (44a) und der Arzneimittelstudiendatenbank (44b) ausgelesen werden.

Es ist denkbar, dass die Arzneimitteldatenbank (44a) eine kombinierte Datenbank ist, bei der eine Arzneimitteldatenbank und eine Verifikationsdatenbank in einer Datenbank vereint sind. Es ist denkbar, dass die Steuereinheit (43) des zweiten Computers (40) konfiguriert ist, zu verifizieren, dass es sich bei der Einzelpackung um eine legal erworbene, originale Einzelpackung handelt.

Figur 8 zeigt schematisch in einem Ablaufdiagramm eine Ausführungsform des erfindungsgemäßen Verfahrens, das mit dem in Figur 7 gezeigten System ausgeführt werden kann.

Das Verfahren (300) umfasst die Schritte:
(310) Erfassen eines maschinenlesbaren, zweidimensionalen Codes eines individuellen Erkennungsmerkmals auf einer Einzelpackung eines Arzneimittels mit Hilfe eines Lesegerätes durch einen potenziellen Teilnehmer einer Arzneimittelstudie an einem ersten Computer
(320) Ermitteln eines Produktcodes und einer Seriennummer aus dem zweidimensionalen Code
(330) Übertragen des Produktcodes und der Seriennummer vom ersten Computer an einen zweiten Computer
(340) Ermitteln eines Arzneimittels und/oder einer Indikation anhand des übertragenen Produktcodes und mit Hilfe einer Arzneimitteldatenbank, in der Informationen über das Arzneimittel unter dem Produktcode gespeichert sind
(350) Identifizieren einer oder mehrerer Arzneimittelstudien, bei denen das ermittelte Arzneimittel und/oder die ermittelte Indikation ein Untersuchungsgegenstand sind, mit Hilfe einer Arzneimittelstudiendatenbank
(360) Übertragen der einen oder mehreren identifizierten Arzneimittelstudien vom zweiten Computer an den ersten Computer
(370) Anzeigen der übertragenen einen oder mehreren identifizierten Arzneimittelstudien am ersten Computer gegenüber dem potenziellen Teilnehmer.

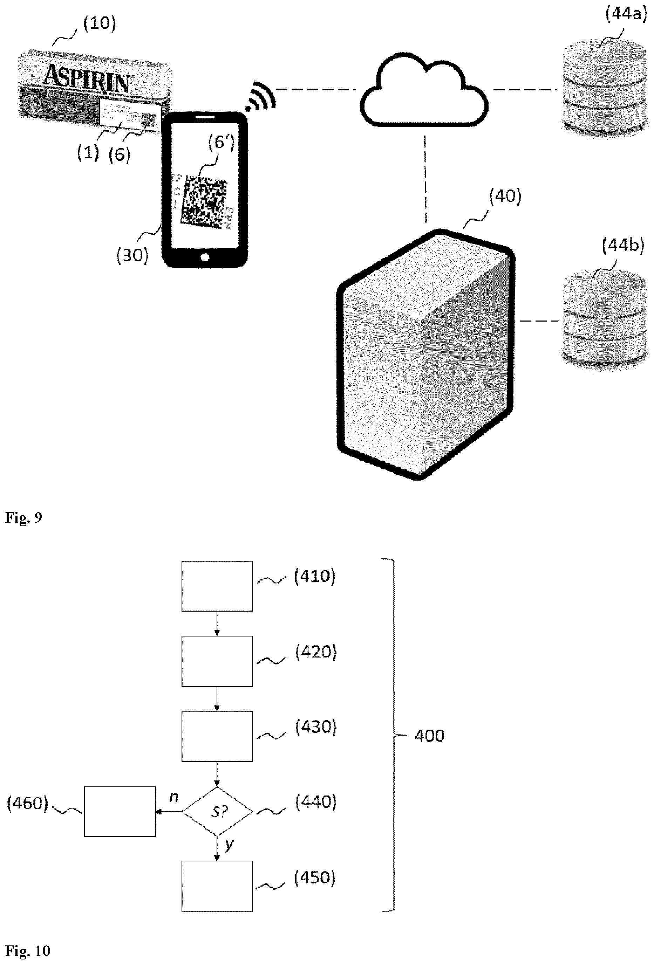

Figur 9 zeigt beispielhaft eine Ausführungsform für das in Figur 7 gezeigte erfindungsgemäße System zusammen mit der in Figur 2 gezeigten Einzelpackung (10) eines Arzneimittels mit einem individuellen Erkennungsmerkmal (1), das einen maschinenlesbaren zweidimensionalen Code (6) trägt.

Der erste Computer (30) ist als ein Smartphone ausgeführt. Die in einem Smartphone üblicherweise vorhandene Kamera wird als Lesegerät für den maschinenlesbaren zweidimensionalen Code verwendet. Mit der Kamera wird eine Abbildung (6') des maschinenlesbaren zweidimensionalen Codes erzeugt und auf einem Bildschirm des Smartphones angezeigt. Das Smartphone ist so konfiguriert, dass es den zweidimensionalen Code dekodieren kann. Dabei werden der Produktcode und die Seriennummer identifiziert. Diese Daten können über ein Mobilfunknetz und das Internet (dargestellt durch die Cloud) an den zweiten Computer (40) übermittelt werden. Der zweite Computer (40) ist als Server ausgeführt. Der Server ist über verschiedene Netzwerke mit der Arzneimitteldatenbank (44a) und der Arzneimittelstudiendatenbank (44b) verbunden. Anhand des Produktcodes kann der Server in der Arzneimitteldatenbank (44a) Informationen zum Arzneimittel und der Indikation, in der es verwendet wird, ermitteln. Anhand des ermittelten Arzneimittels oder der ermittelten Indikation kann der Server in der Arzneimittelstudiendatenbank (44b) eine oder mehrere Arzneimittelstudien identifizieren, die die Indikation und/oder das Arzneimittel zum Gegenstand hat/haben. Informationen zu der Arzneimittelstudie können vom Server über das Internet und das Mobilfunknetz auf das Smartphone übertragen und dort auf dem Bildschirm gegenüber einem potenziellen Teilnehmer angezeigt werden.

Es ist denkbar, dass die Arzneimitteldatenbank (44a) eine kombinierte Datenbank ist, bei der eine Arzneimitteldatenbank und eine Verifikationsdatenbank in einer Datenbank vereint sind. Es ist denkbar, dass die Steuereinheit (43) des zweiten Computers (40) konfiguriert ist, zu verifizieren, dass es sich bei der Einzelpackung um eine legal erworbene, originale Einzelpackung handelt.

Figur 10 zeigt schematisch in einem Ablaufdiagramm eine weitere Ausführungsform des erfindungsgemäßen Verfahrens, das mit einem der in den Figuren 3, 5, 7 und 9 gezeigten Systemen ausgeführt werden kann.

Das Verfahren (400) umfasst die Schritte:
(410) Erfassen eines individuellen Erkennungsmerkmals, das in Form eines zweidimensionalen optisch lesbaren Codes auf einer Einzelpackung eines Arzneimittels aufgebracht ist, mittels einer Kamera durch einen potenziellen Teilnehmer einer Arzneimittelstudie,
(420) Extrahieren eines Produktcodes und einer Seriennummer aus dem erfassten individuellen Erkennungsmerkmals,
(430) Ermitteln eines Arzneimittels aus einer Arzneimitteldatenbank anhand des Produktcodes,
(440) Prüfen ob für den Productcode und/oder das ermittelte Arzneimittel in einer Arzneimittelstudiendatenbank eine geplante oder laufende Arzneimittelstudie (S) gespeichert ist,
(450) für den Fall, dass für den Productcode und/oder das ermittelte Arzneimittel in der Arzneimittelstudiendatenbank eine geplante oder laufende Arzneimittelstudie (S) gespeichert ist: Anzeigen von Informationen zu der Arzneimittelstudie gegenüber dem potenziellen Teilnehmer,
(460) für den Fall, dass für den Productcode und das ermittelte Arzneimittel in der Arzneimittelstudiendatenbank keine geplante und keine laufende Arzneimittelstudie (S) gespeichert ist: Anzeigen einer Mitteilung gegenüber dem potenziellen Teilnehmer, dass aktuell für das Arzneimittel keine Arzneimittelstudie läuft und geplant ist.

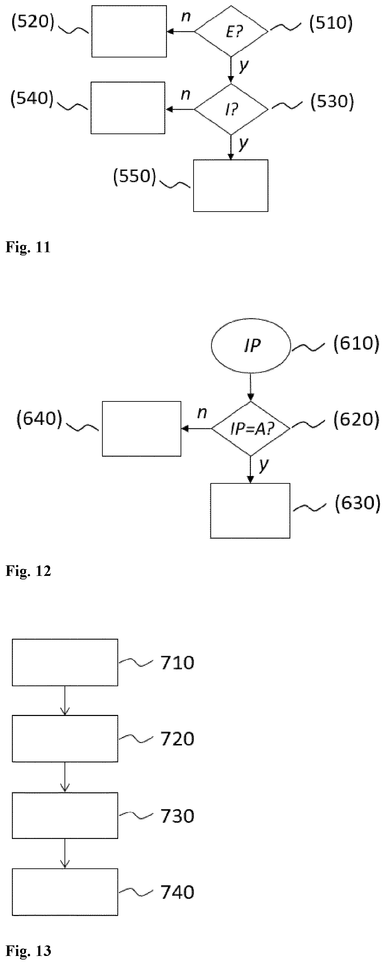

Figur 11 zeigt schematisch in einem Ablaufdiagramm weitere optionale Schritte des erfindungsgemäßen Verfahrens, das mit einem der in den Figuren 3, 5, 7 und 9 gezeigten Systemen ausgeführt werden kann.

Die Schritte sind:
(510) Prüfen ob für den Produktcode und die Seriennummer ein Eintrag (E) in einer Verifikationsdatenbank existiert,
(520) für den Fall, dass in der Verifikationsdatenbank kein Eintrag (E) für den Produktcode und die Seriennummer existiert: Anzeigen einer Mitteilung gegenüber dem potenziellen Teilnehmer, dass eine Registrierung für eine Arzneimittelstudie nicht möglich ist,
(530) für den Fall, dass in der Verifikationsdatenbank ein Eintrag (E) für den Produktcode und die Seriennummer existiert: Prüfen, ob der Eintrag eine Information (I) umfasst, dass das Arzneimittel als abgegeben gekennzeichnet ist,
(540) für den Fall, dass das Arzneimittel nicht als abgegeben gekennzeichnet ist: Anzeigen einer Mitteilung gegenüber dem potenziellen Teilnehmer, dass eine Registrierung für eine Arzneimittelstudie nicht möglich ist,
(550) für den Fall, dass das Arzneimittel als abgegeben gekennzeichnet ist: Anzeigen einer Mitteilung gegenüber dem potenziellen Teilnehmer, dass eine Registrierung für eine Arzneimittelstudie möglich ist.

Die Schritte (510) bis (550) können beispielsweise nach Schritt (330) oder (350) oder (420) oder (450) erfolgen.

Figur 12 zeigt schematisch in einem Ablaufdiagramm weitere optionale Schritte des erfindungsgemäßen Verfahrens, das mit einem der in den Figuren 3, 5, 7 und 9 gezeigten Systemen ausgeführt werden kann.

Die Schritte sind:
(610) Empfangen von Informationen (IP) über den potenziellen Teilnehmer von dem potenziellen Teilnehmer, wobei die Informationen Alter, Geschlecht, Körpergröße, Körpergewicht, Krankheitssymptome, diagnostizierte Erkrankung und/oder Vorerkrankungen umfassen,
(620) Vergleichen der Informationen über den potenziellen Teilnehmer mit in der Arzneimittelstudiendatenbank gespeicherten Anforderungen (A) für eine Arzneimittelstudie,
(630) für den Fall, dass die Informationen (IP) über den potenziellen Teilnehmer die Anforderungen (A) erfüllen: Anzeigen von Informationen zu der Arzneimittelstudie gegenüber dem potenziellen Teilnehmer und/oder Auffordern des potenziellen Teilnehmers zum Registrieren für die Arzneimittelstudie,
(640) für den Fall, dass die Informationen (IP) über den potenziellen Teilnehmer die Anforderungen (A) nicht erfüllen: Anzeigen einer Mitteilung gegenüber dem potenziellen Teilnehmer, dass keine Arzneimittelstudie läuft oder geplant ist, für die er sich registrieren kann.

Die Schritte (610) bis (640) können beispielsweise nach Schritt (240) oder (370) oder (450) oder (550) erfolgen.

Figur 13 zeigt schematisch in einem Ablaufdiagramm weitere optionale Schritte des erfindungsgemäßen Verfahrens, das mit einem der in den Figuren 3, 5, 7 und 9 gezeigten Systemen ausgeführt werden kann.

Die Schritte sind:
(710) Erzeugen einer individuellen Kennung für den potenziellen Teilnehmer unter Verwendung des Productcodes und/oder der Seriennummer,
(720) Erfassen des potenziellen Teilnehmer als Teilnehmer der Arzneimittelstudie unter der individuellen Kennung in einer Datenbank,
(730) Empfangen von Gesundheitsdaten des Teilnehmers der Arzneimittelstudie während der Arzneimittelstudie
(740) Speichern der Gesundheitsdaten unter der individuellen Kennung in der Datenbank.

Die Schritte (710) bis (740) können beispielsweise nach Schritt (370) oder (450) oder (550) oder (630) erfolgen.

## Patentansprüche

1. System umfassend
- eine Eingabeeinheit,
- eine Ausgabeeinheit,
- eine Steuereinheit und
- eine Datenbank,
wobei die Steuereinheit konfiguriert ist, die Eingabeeinheit zu veranlassen, Daten eines individuellen Erkennungsmerkmals, das auf einer Einzelpackung eines Arzneimittels angebracht ist, von einem potenziellen Teilnehmer einer Arzneimittelstudie zu empfangen,
wobei die Steuereinheit konfiguriert ist, anhand der empfangenen Daten eine oder mehrere Arzneimittelstudien in der Datenbank zu identifizieren, wobei das Identifizieren der einen oder der mehreren Arzneimittelstudien in der Datenbank umfasst:
- Extrahieren eines Produktcodes und einer Seriennummer aus den Daten des individuellen Erkennungsmerkmals,
- Verifizieren, dass es sich bei der Einzelpackung um eine originale und/oder legal erworbene Einzelpackung handelt, anhand des Produktcodes und der Seriennummer,
- Identifizieren eines Arzneimittels und/oder einer Indikation anhand des Produktcodes,
- Identifizieren einer oder mehrerer Arzneimittelstudien anhand des identifizierten Arzneimittels und/oder der identifizierten Indikation,
wobei die Steuereinheit konfiguriert ist, die Ausgabeeinheit zu veranlassen, Informationen zu der einen oder den mehreren identifizierten Arzneimittelstudien gegenüber dem potenziellen Teilnehmer anzuzeigen, wobei dem potenziellen Teilnehmer Informationen zu einer oder mehreren Arzneimittelstudien nur dann angezeigt wird/werden, wenn es sich bei der Einzelpackung um eine originale und/oder legal erworbene Einzelpackung handelt.

2. System gemäß Anspruch 1,
wobei die Steuereinheit konfiguriert ist, die Eingabeeinheit zu veranlassen, Patienteninformationen von dem potenziellen Teilnehmer zu empfangen,
wobei das Identifizieren der einen oder der mehreren Arzneimittelstudien unter Verwendung der Patienteninformationen erfolgt.

3. System gemäß Anspruch 1 oder 2
wobei die Steuereinheit konfiguriert ist, die Eingabeeinheit zu veranlassen,
- ein Portal zum Anmelden des potenziellen Teilnehmers bereitzustellen, wobei der potenzielle Teilnehmer über das Portal Daten des individuellen Erkennungsmerkmals und Patienteninformationen in den Computer eingibt, und dem potenziellen Teilnehmer über das Portal Informationen zu einer oder zu mehreren Arzneimittelstudien angezeigt werden.

4. System gemäß Anspruch 3,
wobei die Steuereinheit konfiguriert ist, den potenziellen Teilnehmer als einen Teilnehmer einer identifizierten Arzneimittelstudie zu registrieren,
wobei die Steuereinheit konfiguriert ist, Ergebnisse der Arzneimittelstudie, für die der Teilnehmer registriert ist, über das Portal, insbesondere durch Eingabe von Informationen zum Gesundheitszustand des Teilnehmers durch den Teilnehmer zu erfassen.

5. System gemäß Anspruch 1 umfassend
- einen ersten Computer mit einem Lesegerät und einer Ausgabeeinheit, und
- einen zweiten Computer, der mit einer Arzneimitteldatenbank, einer Arzneimittelstudiendatenbank und einer Verifikationsdatenbank verbunden ist,
wobei der erste Computer konfiguriert ist,
- einen maschinenlesbaren zweidimensionalen Code eines individuellen Erkennungsmerkmals mit Hilfe des Lesegeräts zu erfassen,
- aus dem Code einen Produktcode und eine Seriennummer zu extrahieren,
- den Produktcode und die Seriennummer über ein Netzwerk an den zweiten Computer zu übermitteln,
wobei der zweite Computer konfiguriert ist,
- den Produktcode und die Seriennummer über das Netzwerk von dem ersten Computer zu empfangen,
- zu verifizieren, dass in der Verifikationsdatenbank Informationen zu einer Einzelpackung eines Arzneimittels gespeichert sind, wobei der Einzelpackung der Produktcode und die Seriennummer zugeordnet sind,
- anhand des Produktcodes ein Arzneimittel und/oder eine Indikation in der Arzneimitteldatenbank zu identifizieren,
- anhand des identifizierten Arzneimittels und/oder der identifizierten Indikation ein oder mehrere Arzneimittelstudien in der Arzneimittelstudiendatenbank zu identifizieren,
- Informationen zu der einen oder den mehreren identifizierten Arzneimittelstudien über das Netzwerk an den ersten Computer zu übermitteln,
wobei der erste Computer konfiguriert ist,
- die Informationen zu der einen oder den mehreren identifizierten Arzneimittelstudien über das Netzwerk von dem zweiten Computer zu empfangen,
- die Informationen zu der einen oder den mehreren identifizierten Arzneimittelstudien über die Ausgabeeinheit anzuzeigen.

6. System gemäß Anspruch 5,
wobei der erste Computer konfiguriert ist, Patienteninformationen und/oder Informationen zur Selbsteinschätzung des Gesundheitszustands eines Teilnehmers einer Arzneimittelstudie über die Eingabeeinheit zu empfangen und an den zweiten Computer über ein Netzwerk zu übermitteln.

7. Computerprogrammprodukt umfassend einen Datenträger und Programmcode, der auf dem Datenträger gespeichert ist, und der einen Computer, in dessen Arbeitsspeicher der Programmcode geladen ist, dazu veranlasst, die folgenden Schritte auszuführen:
- Empfangen von Daten eines individuellen Erkennungsmerkmals, das auf einer Einzelpackung eines Arzneimittels aufgebracht ist,
- Identifizieren einer oder mehrerer Arzneimittelstudien anhand der empfangenen Daten, wobei das Identifizieren der einen oder der mehreren Arzneimittelstudien in der Datenbank umfasst:
o Extrahieren eines Produktcodes und einer Seriennummer aus den Daten des individuellen Erkennungsmerkmals,
o Verifizieren, dass es sich bei der Einzelpackung um eine originale und/oder legal erworbene Einzelpackung handelt, anhand des Produktcodes und der Seriennummer,
o Identifizieren eines Arzneimittels und/oder einer Indikation anhand des Produktcodes,
o Identifizieren einer oder mehrerer Arzneimittelstudien anhand des identifizierten Arzneimittels und/oder der identifizierten Indikation,
- Anzeigen von Informationen über die eine oder die mehreren Arzneimittelstudien gegenüber einem potenziellen Teilnehmer der einen oder der mehreren Arzneimittelstudien, wobei dem potenziellen Teilnehmer eine oder mehrere Arzneimittelstudien nur dann angezeigt wird/werden, wenn es sich bei der Einzelpackung um eine originale und/oder legal erworbene Einzelpackung handelt.

8. Computerprogrammprodukt gemäß Anspruch 7, wobei der Programmcode den Computer ferner dazu veranlasst, den folgenden Schritt auszuführen:
- Empfangen von Patienteninformationen durch den potenziellen Teilnehmer in den Computer,
wobei das Identifizieren der einen oder der mehreren Arzneimittelstudien unter Verwendung der Patienteninformationen erfolgt.

9. Computerprogrammprodukt gemäß einem der Ansprüche 7 oder 8, wobei der Programmcode den Computer ferner dazu veranlasst, den folgenden Schritt auszuführen:
- Bereitstellen eines Portals zum Anmelden von Teilnehmern an einer Arzneimittelstudie auf dem Computer, wobei der Computer durch den potenziellen Teilnehmer bedient wird, wobei der potenzielle Teilnehmer über das Portal Daten des individuellen Erkennungsmerkmals und Patienteninformationen in den Computer eingibt, und dem potenziellen Teilnehmer über das Portal Informationen zu einer oder zu mehreren Arzneimittelstudien angezeigt werden.

10. Computerprogrammprodukt gemäß Anspruch 9, wobei der Programmcode den Computer ferner dazu veranlasst, die folgenden Schritte auszuführen:
- Registrieren des potenziellen Teilnehmers als einen Teilnehmer einer identifizierten Arzneimittelstudie,
- Erfassen von Ergebnissen der Arzneimittelstudie, für die der Teilnehmer registriert ist, über das Portal, insbesondere durch Eingabe von Informationen zum Gesundheitszustand des Teilnehmers durch den Teilnehmer.

11. Computerprogrammprodukt gemäß Anspruch 11, wobei der Teilnehmer bei der Registrierung für die Arzneimittelstudie mit einer individuellen Kennung verknüpft wird, die aus einer Kombination aus Produktcode und Seriennummer des individuellen Erkennungsmerkmals abgeleitet ist.

12. Verwendung eines individuellen Erkennungsmerkmals, das auf einer Einzelpackung eines Arzneimittels angebracht ist, zur Rekrutierung von Teilnehmern für eine Arzneimittelstudie, **dadurch gekennzeichnet, dass** ein potenzieller Teilnehmer Daten des individuellen Erkennungsmerkmals in einen Computer eingibt, anhand der Daten eine oder mehrere Arzneimittelstudien identifiziert werden,
wobei das Identifizieren der einen oder der mehreren Arzneimittelstudien in der Datenbank umfasst:
o Extrahieren eines Produktcodes und einer Seriennummer aus den Daten des individuellen Erkennungsmerkmals,
o Verifizieren, dass es sich bei der Einzelpackung um eine originale und/oder legal erworbene Einzelpackung handelt, anhand des Produktcodes und der Seriennummer,
o Identifizieren eines Arzneimittels und/oder einer Indikation anhand des Produktcodes,
o Identifizieren einer oder mehrerer Arzneimittelstudien anhand des identifizierten Arzneimittels und/oder der identifizierten Indikation,
und dem potenziellen Teilnehmer Informationen zu der einen oder den mehreren Arzneimittelstudien angezeigt werden, wobei dem potenziellen Teilnehmer Informationen zu der einen oder den mehreren Arzneimittelstudien nur dann angezeigt wird/werden, wenn es sich bei der Einzelpackung um eine originale und/oder legal erworbene Einzelpackung handelt.

13. Verwendung gemäß Anspruch 12, wobei die Eingabe der Daten des individuellen Erkennungsmerkmals in den Computer über ein von dem Computer bereitgestelltes Portal zum Anmelden von Teilnehmern an einer Arzneimittelstudie auf dem Computer erfolgt, wobei der Computer durch den potenziellen Teilnehmer bedient wird, wobei der potenzielle Teilnehmer über das Portal Daten des individuellen Erkennungsmerkmals und Patienteninformationen in den Computer eingibt, und dem potenziellen Teilnehmer über das Portal Informationen zu einer oder zu mehreren Arzneimittelstudien angezeigt werden.

14. Verwendung gemäß Anspruch einem der Ansprüche 12 oder 13, wobei der potenzielle Teilnehmer als ein Teilnehmer einer identifizierten Arzneimittelstudie anhand des individuellen Erkennungsmerkmals registriert wird.

15. Verwendung gemäß Anspruch 14, wobei Ergebnisse der Arzneimittelstudie, für die der Teilnehmer registriert ist, über das Portal, insbesondere durch Eingabe von Informationen zum Gesundheitszustand des Teilnehmers durch den Teilnehmer, erfasst werden.
